# EUROPÄISCHE PATENTSCHRIFT

(11) **EP 0 413 188 B1**
(45) Veröffentlichungstag und Bekanntmachung des Hinweises auf die Patenterteilung: **01.02.1995**
(21) Anmeldenummer: 90114576.3
(22) Anmeldetag: 30.07.1990
(51) Int. Cl.: A61K 39/395

(54) **Verfahren zur Herstellung nicht modifizierter intravenös verabreichbarer IgM- und/oder IgA-haltige Immunglobulinpräparate**
Method for producung an unmodified intravenous IgM- and/or IgA-containing immunoglobulin preparation
Procédé de préparation d'immunogluboline, non-modifiée contenant des IgM et/ou des IgA, administrables par voie intraveineuse

(30) Priorität: 17.08.1989 DE 3927111
(43) Veröffentlichungstag der Anmeldung: 20.02.1991
(73) Patentinhaber: BIOTEST PHARMA GMBH, D-63303 Dreieich (DE)
(72) Erfinder: Möller, Wolfgang, Dipl.Chem. Dr., D-6370 Oberursel (DE); Piechaczek, Detlef, Dipl.Chem. Dr., D-6115 Münster (DE)
(74) Vertreter: Beil, Hans Chr., Dr.

(56) Entgegenhaltungen:
- EP-A- 0 013 901
- EP-A- 0 268 973
- EP-A- 0 352 500
- DE-A- 2 404 265
- DE-A- 3 310 150
- FR-A- 2 314 730
- FR-A- 2 433 342
- US-A- 3 808 189

## Beschreibung

Die Erfindung betrifft ein Verfahren zur Herstellung intravenös verträglicher Immunglobulin-Präparate, die chemisch nicht modifiziert sind und mehr als 5% IgM und/oder mehr als 10% IgA, bezogen auf den Gesamtimmunglobulingehalt, enthalten.

Die Immunglobuline sind eine Gruppe von Glykoproteinen, die im Körper als Antwort auf das Auftauchen fremder Antigenen gebildet werden und die für deren Bindung und anschließende Eliminierung zuständig sind. Man unterscheidet mehrere Klassen von Immunglobulinen, wovon aber normalerweise nur IgG, IgA und IgM in höheren Konzentrationen im Plasma gemessen werden.

IgG ist mit ca. 12 mg/ml das Hauptimmunglobulin im Plasma, und hauptsächlich für die Abwehr viraler Infektionen zuständig. Die Konzentration für IgM liegt mit ca. 1,5 mg/ml deutlich niedriger. IgM ist vorwiegend für die Abwehr von bakteriellen Erregern und für die Bindung von Bakterien-Toxinen verantwortlich. IgA ist mit einer durchschnittlichen Plasmakonzentration von 3,5 mg/ml für die Neutralisation von verschiedenen Viren, wie z.B. Poliomyelitis, Masern und Influenza zuständig und in seiner sekretorischen, dimeren Form vor allem in seromukosen Sekreten vertreten.

Für die passive Immuntherapie werden seit nunmehr 40 Jahren Immunglobulin-Präparate eingesetzt. Dabei handelt es sich zum überwiegenden Teil um reine IgG-Präparationen mit nur sehr geringem IgA- und IgM-Gehalt. Bis zum Beginn der sechziger Jahre waren die Immunglobulin-Präparate zudem nur intramuskulär zu applizieren, was wegen der schmerzhaften Nebenwirkungen die Gabe größerer Mengen unmöglich machte.

In der folgenden Zeit wurden dann mehrere intravenös verträgliche IgG-Präparationen entwickelt, indem man das IgG entweder chemisch oder enzymatisch modifizierte oder durch andere Methoden iv-verträglich machte.

IgG-Präparate, die neben dem IgG noch nennenswerte Mengen IgM und/oder IgA enthielten, wie z.B. in der DE-PS 2404265 oder der US-PS 3808189 beschrieben, waren aber weiterhin nur intramuskulär verträglich.

Die ersten und bisher einzigen IgM- und/oder IgA-haltigen intravenös verträglichen Immunglobulin-Präparate sind in der EP-PS 0013901 und der DE-OS 3825429, entsprechend der EP-A 0 352 500, beschrieben. Die iv-Verträglichkeit wird in diesen beiden Präparaten im wesentlichen durch eine chemische Modifizierung mit β-Propiolakton erreicht. Ein Maß für die iv-Verträglichkeit von Immunglobulinpräparaten ist die Antikomplementäre Aktivität (ACA) gemessen nach Kabat, E. and Mayer, M. in Experimental Immunochemistry 2nd ed., Thomas Brooks, Springfield, II, 133-240 (1964).

Die EP-A 0 268 973 offenbart ein Verfahren zur Herstellung eines iv-verträglichen IgG-Präparates, welches durch Ionenaustauscher-Chromatographie und durch Affinitäts-Chromatographie und/oder Antithrombin-III-Behandlung erhalten wird.

Die EP-A 0 303 088 betrifft ein gereinigtes IgM-Produkt durch Ionenaustausch- und Gelchromatographie, welches ausschließlich aus monoklonalen Antikörpern besteht.

Der Erfindung lag die Aufgabe zu Grunde, IgM- und/oder IgA-haltige Immunglobulinpräparate zu entwickeln, die eine niedrige ACA haben und intravenös verträglich, aber chemisch nicht modifiziert sind.

Die Aufgabe wurde dadurch gelöst, daß man eine IgM- und/oder IgA-haltige Immunglobulinlösung an einen Anionenaustauscher bindet, durch Gradientenelution eine Fraktion mit einer ACA kleiner 50 CH50/ml bei 50 g Immunglobulin pro Liter Lösung eluiert und diese Fraktion gegebenenfalls einer kurzzeitigen Behandlung bei niedrigem pH-Wert und/oder bei erhöhter Temperatur unterzieht. Die Elution erfolgt dabei mit einem Puffer im sauren pH-Bereich.

Überraschenderweise wurde gefunden, daß bei der Anionenaustauschchromatographie die Fraktion, die für die hohe ACA, insbesondere größer 50 CH50/ml bei 50 g Immunglobulin pro Liter Lösung verantwortlich ist, so fest gebunden wird, daß durch geeignete Elutionsbedingungen rund 95% der aufgetragenen Immunglobuline einschließlich der Hauptmenge des IgM und des IgA mit niedriger ACA eluiert werden können.

Als Anionenaustauscher eignen sich vorzugsweise TMAE-, QMA- oder DEAE-Gruppen tragende Polymere, wie z.B. DEAE-Trisacryl^{(R)} (Copolymer aus N-Acryloyl-2-amino-hydroxy-methyl-1,3-propandiol und dem Diethylaminomethylderivat hiervon), QAE-Trisacryl^{(R)} (Copolymer aus N-Acryloyl-2-amino-2-hydroxyl-methyl-1,3-propandiol und dem Trimethylaminomethylderivat hiervon), alpha-Sepharose(R) bzw. Mono-Q^{(R)} (neutrale Polysaccharide mit quaternären Aminogruppen).

Verwendet man ein Ausgangsmaterial mit erhöhter ACA, kann man durch zusätzliche kurzzeitige Behandlung dieses Eluates bei niedrigem pH oder erhöhter Temperatur die ACA auf für iv-verträgliche Produkte normale Werte reduzieren.

Es versteht sich von selbst, daß das Ausgangsmaterial für die Anionaustauschchromatographie immer unter möglichst Protein-schonenden Bedingungen hergestellt werden muß. Als Ausgangsmaterialien eignen sich Immunglobulin-haltige Lösungen, wie zum Beispiel die Cohn-Fraktion II/III oder III oder andere IgA- und/oder IgM-haltige Plasmafraktionen oder andere Lösungen, wie zum Beispiel Milch.

Zum Beispiel kann man eine Immunglobulin-haltige Fraktion, wie die Cohn-Fraktion II/III oder III, in Puffer lösen, und die meisten Verunreinigungen durch eine Fällung mit 0,5 bis 5% Oktansäure bei pH 4 bis 6, vorzugsweise pH 5, entfernen. Anschließend wird die Lösung bei niedriger Konduktivität mit einem Anionenaustauscher behandelt, so daß die Hauptmenge des IgA und des IgM gebunden werden. Die Adsorption kann dabei sowohl im Batch-Verfahren, wie auch in einer Chromatographie-Säule oder an Membranen durchgeführt werden.

Soll das gewünschte Produkt IgA und IgM enthalten, wird mit einem Salzgradienten derart eluiert, daß ca. 10 bis 20% des IgM auf der Matrix adsorbiert bleiben. Die exakten Elutionsbedingungen sind von der Art des Anionenaustauschers abhängig. Sie bewegen sich aber in dem Bereich von 10 bis 400 mOsmol je nach Matrix und pH-Wert. Einzelheiten für ausgewählte Träger sind in den Beispielen beschrieben.

Soll das Produkt nur IgA, aber kein IgM enthalten, wird mit einer niedrigeren Osmolarität eluiert, so daß das IgM an der Matrix adsorbiert bleibt. Je nach Chromatographiebedingungen enthält das Eluat 30 bis 60% IgA neben 70 bis 40% IgG, insbesondere 10-35 Gew.-% IgM und 10 bis 35 Gew.-% IgA und ganz besonders 5-100 Gew.-% IgM, bezogen auf den Gesamtimmunglobulingehalt. Die ACA ist in solch einem Produkt auch ohne weitere Behandlung sehr niedrig. Durch geeignete Maßnahmen kann das IgA weiter aufgereinigt werden.

Enthält das Eluat dagegen auch nennenswerte Mengen IgM, kann durch eine zusätzliche kurzzeitige Behandlung für 1 Minute bis 24 Stunden bei niedrigem pH, vorzugsweise pH 4 bis 4,5 und/oder bei erhöhter Temperatur, bei 40 bis 60° C, vorzugsweise bei 50 bis 54° C, die ACA weiter reduziert werden.

Eine reine IgM-Lösung, weitgehend befreit von IgA läßt sich gewinnen, indem man den Anionenaustauscher mit Puffer derart vorwäscht, daß das IgA vor dem IgM eluiert wird.

Bei niedriger ACA im Ausgangsmaterial kann unter Umständen die gesamte auf dem Anionenaustauscher adsorbierte IgM-Fraktion eluiert werden. In diesem Fall reicht eine kurzzeitige Behandlung des Eluats für 1 Minute bis 4 Stunden bei niedrigem pH, vorzugsweise pH 4 bis 4,5 und/oder bei erhöhter Temperatur, bei 40 bis 60° C, vorzugsweise bei 50 bis 54° C alleine aus, um die ACA auf verträgliche Maße zu reduzieren.

Durch Ultra- und Diafiltration kann die Lösung dann konzentriert und im Elektrolytgehalt auf die endgültige iv-Formulierung eingestellt werden. Die ACA der Endprodukte liegt dann in einem Bereich, wie sie für marktübliche iv-IgG-Präparate oder für das chemisch modifizierte IgM-Präparat Pentaglobin üblich sind. Die Proteinkonzentration kann 1 bis 20 g/100 ml, vorzugsweise 3-5 g/100 ml betragen. Das Präparat kann zusätzlich Proteine, vorzugsweise Humanalbumin, Zucker, vorzugsweise Glucose, oder Aminosäuren enthalten.

Da die ACA der vom Anionenaustauscher nicht gebundenen IgG-Fraktion ebenfalls sehr niedrig ist, kann man aus dieser IgG-Fraktion und den erfindungsgemäßen IgA- und/oder IgM-haltigen Fraktionen Mischungen herstellen, um zu iv-verträglichen Immunglobulinpräparaten mit niedriger ACA und einer gewünschten Verteilung an IgG, IgA und IgM zu gelangen. So läßt sich zum Beispiel ein IgM- und IgA-haltiges Immunglobulin-Präparat mit der gleichen Zusammensetzung wie das Handelsprodukt Pentaglobin mit 80% IgG, 10% IgA und 10% IgM herstellen, wobei die ACA gleich hoch oder sogar niedriger ist, als bei dem durch chemische Modifizierung erhaltenen Pentaglobin.

Die erfindungsgemäßen IgM- und/oder IgA-haltigen Immunglobulinpräparate können vor oder nach den erfindungsgemäßen Verfahrensschritten an sich bekannten Sterilisationsverfahren, wie β-Propiolakton,insbesondere mit 0,05%/UV-Behandlung, der Behandlung mit Lösungsmitteln und/oder Detergentien oder der Pasteurisation, unterzogen werden.

Die folgenden Beispiele erläutern die Erfindung ohne sie auf sie zu beschränken:

### Beispiel 1:

10 kg Cohn-Paste III wurden in 50 l 0,1 M Acetatpuffer, pH 5 gelöst und mit 1,5 kg Oktansäure bei 25° C versetzt. Der Niederschlag wurde nach 4 Stunden abzentrifugiert und der Überstand gegen 20 mM Piperazin, 20 mM NaCl, pH 6 diafiltriert. Die Lösung wurde anschließend auf eine 5 l Säule mit TMAE-Fraktogel^{(R)} (Firma Merck, Darmstadt), die mit dem gleichen Puffer äquilibriert war, in 5 Chromatographie-Läufen aufgetragen. Die IgG-Fraktion, die dabei nicht gebunden wurde, wurde gesammelt und durch Ultrafiltration konzentriert.

Mit 20 mM Piperazin, 100 mM NaCl, pH 6 ließ sich eine IgA-reiche Fraktion und anschließend mit 20 mM Piperazin, 150 mM NaCl, pH 6 eine IgM-reiche Fraktion eluieren. Mit 20 mM Piperazin, 190 mM NaCl, pH 6 wurde dann das restliche gebundene Protein von der Säule gewaschen. Tabelle 1 gibt die Zusammensetzung und die ACA der Fraktion wieder.

**Tabelle 1**

| | IgG g/l | IgA g/l | IgM g/l | ACA CH 50/ml |
|---|---|---|---|---|
| IgG-Fraktion | 50 | 0,3 | 0 | 9 |
| IgA-Fraktion | 31 | 18 | 0,1 | 17 |
| IgM-Fraktion | 9 | 9 | 31 | 41 |
| Restfraktion | 8 | 3 | 39 | 441 |

Aus den IgG-, IgA- und IgM-Fraktionen dieses Beispiels wurde durch entsprechendes Mischen ein chemisch unmodifiziertes Immunglobulinpräparat mit der Zusammensetzung 80% IgG, 10% IgA und 10% IgM hergestellt und mit dem β-Propiolakton-modifizierten Handelsprodukt Pentaglobin verglichen (Tabelle 2).

**Tabelle 2**

| | IgG g/l | IgA g/l | IgM g/l | ACA CH 50/ml |
|---|---|---|---|---|
| unmodifiziertes Präparat (erfindungsgemäß) | 40,0 | 4,9 | 5,0 | 13 |
| Pentaglobin Lot 1462019 (Vergleich) | 43,4 | 4,2 | 5,0 | 26 |

### Beispiel 2:

1 kg Cohn-Paste III wurde analog zu Beispiel 1 aufgearbeitet. Nach dem Auftragen auf die Chromatographie-Säule wurde gleich mit 20 mM Piperazin, 160 mM NaCl, pH 6 eluiert. Die Fraktion enthielt 50% IgG, 23% IgA und 27% IgM bezogen auf den Gesamtimmunglobulingehalt. Die ACA betrug 26 CH 50/ml.

### Beispiel 3:

1 kg Cohn-Paste III wurde analog zu Beispiel 1 behandelt und auf eine 2 l Säule QMA-Accell^{(R)} in 2 Läufen aufgetragen. Nach dem Auftragen auf die Chromatographie-Säule wurde gleich mit 20 mM Piperazin, 20 mM NaCl, pH 4,7 eluiert. Die Fraktion enthielt 38% IgG, 27% IgA und 35% IgM bezogen auf den Gesamtimmunglobulingehalt. Die ACA betrug 50 CH 50/ml. Durch eine Behandlung für 30 Minuten bei pH 4,0 ließ sich die ACA auf 20 CH 50/ml reduzieren.

### Beispiel 4:

10 kg Cohn-Paste II/III wurde analog zu Beispiel 1 aufgearbeitet. Wegen des höheren IgG-Gehaltes der Paste II/III gegenüber der Paste III wurde die Oktansäuremenge auf 0,75 kg reduziert. In der Tabelle 3 sind die Eigenschaften der Eluate wiedergegeben.

**Tabelle 3**

| | IgG g/l | IgA g/l | IgM g/l | ACA CH 50/ml |
|---|---|---|---|---|
| IgG-Fraktion | 49 | 1 | 0 | 4 |
| IgA-Fraktion | 35 | 15 | 0,2 | 7 |
| IgM-Fraktion | 9 | 10 | 30 | 19 |
| Restfraktion | 10 | 4 | 36 | 193 |

### Beispiel 5:

1 kg Cohn-Paste III wurde analog zu Beispiel 1 aufgearbeitet. Nach dem Auftragen auf die Chromatographie-Säule wurde mit 20 mM Piperazin, 120 mM NaCl, pH 6 gewaschen und mit 20 mM Piperazin, 175 mM NaCl, pH 6 eluiert. Das Eluat wurde durch Ultrafiltration konzentriert und durch Diafiltration auf einen Proteinwert von 50 g/l in 75 mM NaCl, 2,5% Glukose, pH 7 eingestellt. Das Produkt wurde sterilfiltriert, für 20 Minuten auf 50° C erhitzt und anschließend gefriergetrocknet. Das Immunglobulin-Präparat hatte die in Tabelle 4 aufgeführten Eigenschaften.

**Tabelle 4**

| IgM-Präparat (erfindungsgemäß) | Protein g/l | IgM g/l | ACA CH 50/ml | rez. Titer ²) Pseudom. aerug. |
|---|---|---|---|---|
| nach der Chromatographie | 54,3 | 50,5 | 230 | 40960 |
| nach 20 Min., 50° C | 54,3 | 50,5 | 29 | 40960 |
| nach der Gefriergetrocknung ¹) | 50,7 | 46,5 | 27 | 40960 |

| Vergleichspräparat: | | | | |
|---|---|---|---|---|
| Pentaglobin Lot 1462019 | 53,3 | 5,0 | 26 | 1280 |

| | | | | |
|---|---|---|---|---|
| ¹) Rekonstitution mit destilliertem Wasser | | | | |
| ²) Der antibakterielle Titer gegen Pseudomonas aeruginosa wurde nach der Methode der passiven Hämagglutination nach Neter, E, Bact. Rev. 20, 166 (1956) bestimmt. | | | | |

### Beispiel 6:

6 kg Cohn-Paste III wurde analog zu Beispiel 1 aufgearbeitet. Nach dem Auftragen auf die Chromatographie-Säule wurde bei jedem der 3 Läufe mit 20 mM Piperazin, 120 mM NaCl, pH 6 gewaschen und mit 20 mM Piperazin, 175 mM NaCl, pH 6 eluiert. Die 3 IgM-Eluate wurden vereinigt und von den erhaltenen 12 l Eluat 3 l analog zu Beispiel 5 aufgearbeitet.

Die restlichen 9 l wurden in einer Rotationsdurchflußapparatur mit 2 x 20 W UV-Lampen bei 600 UPM und einem Durchfluß von 20 l pro Stunde in 1 cm Abstand bestrahlt.

Nach der Konzentrierung durch Ultrafiltration auf 40 g/l Protein wurde die Hälfte der Lösung mit 0,05 % β-Propiolakton (βPl) für 90 Minuten bei pH 7,2 und 25°C behandelt.

Die andere Hälfte der Lösung wurde für 4 Stunden bei 25°C mit 0,3 % Tri-N-Butylphosphat (TNBP) und 1% Tween 80^{(R)} behandelt. Anschließend wurde in beiden Fällen wie in Beispiel 5 weiterverfahren.
Die Präparate wurde auf gleichen Protein- und IgM-Gehalt eingestellt, die ACA und der antibakterielle Titer sind in der folgenden Tabelle 5 wiedergegeben.

**Tabelle 5**

| | ACA CH 50/ml | rez. Titer Pseud. aerug. |
|---|---|---|
| ohne Sterilisation | 32 | 20480 |
| nach UV/βPl | 30 | 20480 |
| nach UV/TNBP-Tween | 31 | 20480 |

Unter den gewählten Bedingungen, die die Gewähr für eine ausreichende Inaktivierung humanpathogener Viren bieten, werden weder die ACA noch die antibakterielle Wirksamkeit des IgM-Präparates nennenswert verändert.
Die erfindungsgemäßen Immunglobulin-Präparate, die sowohl als gegebenenfalls noch zu verdünnende injizierbare Lösungen oder in gefriergetrockneter Form bereitgestellt werden können, können zusätzlich Proteine, wie beispielsweise Humanalbumin, Zucker, wie beispielsweise Glucose, oder Aminosäuren oder geeignete monoklonale Antikörper zugesetzt werden.

## Patentansprüche

1. Verfahren zur Herstellung eines intravenös verabreichbaren polyklonalen, chemisch unmodifizierten Immunglobulin-Präparates, enthaltend mehr als 5 Gew.-% IgM und/oder mehr als 10% IgA, bezogen auf den Gesamtimmunglobulingehalt, sowie eine niedrige antikomplementäre Aktivität aufweisend, dadurch gekennzeichnet, daß man eine Cohn-Fraktion II/III oder III oder äquivalente IgA- und/oder IgM-haltige Plasmafraktionen mit einem Anionenaustauscher behandelt, diesen mit einem Kochsalzgradienten mit einem Puffer im sauren Bereich in einer Weise eluiert, daß Proteine mit einer antikomplementären Aktivität größer 50 CH50/ml bei 50 g Immunglobulin pro Liter Lösung gebunden bleiben und die Immunglobuline im Eluat eine antikomplementäre Aktivität kleiner 50 CH50/ml bei 50 g Immunglobulin pro Liter Lösung aufweisen.

2. Verfahren nach Anspruch 1, dadurch gekennzeichnet, daß der Kochsalzgradient eine Konzentration von 20-175 mM aufweist.

3. Verfahren nach Anspruch 1, dadurch gekennzeichnet, daß als Puffer 20 mM Piperazin verwendet werden.

4. Verfahren nach Anspruch 1, dadurch gekennzeichnet, daß das Eluat einen pH-Wert im Breich von 4,7 bis 6 aufweist.

5. Verfahren nach Anspruch 1-4, dadurch gekennzeichnet, daß das hergestellte Irnunglobulinpräparat 5-100 Gew.-% IgM, bezogen auf den Gesamtimmunglobulingehalt, aufweist.

6. Verfahren nach Anspruch 1-4, dadurch gekennzeichnet, daß das hergestellte Immunglobulinpräparat 10 bis 35 Gew.-% IgM und 10 bis 35 Gew.-% IgA, bezogen auf den Gesamtimmunglobulingehalt, enthält.

7. Verfahren nach einem der Ansprüche 1 bis 6, dadurch gekennzeichnet, daß das hergestellte Immunglobulinpräparat als Lösung mit einer Proteinkonzentration von 1 bis 20 g/100 ml, vorzugsweise 3 bis 5 g/100 ml, vorliegt.

8. Verfahren nach Anspruch 1 bis 7, dadurch gekennzeichnet, daß das hergestellte Immunglobulinpräparat zusätzlich Proteine, vorzugsweise Humanalbumin, Zucker, vorzugsweise Glucose, oder Aminosäuren enthält.

9. Verfahren zur Herstellung von Immunglobulinpräparaten gemäß Anspruch 1 bis 4, dadurch gekennzeichnet, daß man gegebenenfalls vor oder nach der Behandlung mit dem Anionenaustauscher die Lösung für 1 Minute bis 24 Stunden, vorzugsweise 20 bis 40 Minuten, auf 40 bis 60°C, vorzugsweise 50 bis 54°C, erhitzt.

10. Verfahren zur Herstellung von Immunglobulinpräparaten nach Anspruch 1 bis 4, dadurch gekennzeichnet, daß man die Lösung gegebenenfalls vor oder nach der Behandlung mit dem Anionenaustauscher für 1 Minute bis 24 Stunden bei pH 3,5 bis 5, vorzugsweise pH 4 bis 4,5, inkubiert.

11. Verfahren nach einem der Ansprüche 1 bis 10, dadurch gekennezeichnet, daß man als Anionenaustauscher TMAE-Gruppen tragende Polymere verwendet.

12. Verfahren nach einem der Ansprüche 1 bis 10, dadurch gekennzeichnet, daß man als Anionenaustauscher QMA-Gruppen tragende Polymere verwendet.

13. Verfahren nach einem der Ansprüche 1 bis 10, dadurch gekennzeichnet, daß man als Anionenaustauscher ein Copolymer aus dem primären Monomer N-Acryloyl-2-amino-2-hydroxy-methyl-1,3-propandiol und einem Trimethylaminomethylderivat des Monomers oder neutrale Polysaccaride mit quaternären Aminogruppen verwendet.

14. Verfahren nach einem der Ansprüche 1 bis 10, dadurch gekennezeichnet, daß man als Anionenaustauscher DEAE-Gruppen-tragende Polymere verwendet.

15. Verfahren nach Anspruch 14, dadurch gekennzeichnet, daß man als Anionenaustauscher ein Copolymer aus dem primären Monomer N-Acryloyl-2-amino-2-hydroxy-methyl-1,3-propandiol und einem Diethylaminomethylderivat dieses Monomeren verwendet.

16. Verfahren zur Herstellung von Immunglobulinpräparaten nach einem der Ansprüche 1 bis 15, dadurch gekennzeichnet, daß vor oder nach der Behandlung mit dem Anionenaustauscher in der Lösung vorhandene Viren mit 0,05 %β-Propiolakton und/oder UV-Bestrahlung, durch Behandlung mit Lösungsmitteln und/oder Detergentien oder durch Pasteurisation in Lösung inaktiviert werden.

## Claims

1. Process for the production of an intravenously administrable, polyclonal, chemically unmodified immunoglobulin preparation containing more than 5 wt.% IgM and/or more than 10% IgA, related to the total immunoglobulin content, and having a low anticomplementary activity, characterised in that a Cohn fraction II/III or III or equivalent plasma fractions containing IgA and/or IgM are treated with an anion exchanger, these fractions are eluted with a common salt gradient with a buffer in the acidic range in such a manner that proteins with an anticomplementary activity of greater than 50 CH50/ml at 50 g of immunoglobulin per litre of solution remain bound and the immunoglobulins in the eluate have an anticomplementary activity of less than 50 CH50/ml at 50 g of immunoglobulin per litre of solution.

2. Process according to claim 1, characterised in that the common salt gradient has a concentration of 20-175 mM.

3. Process according to claim 1, characterised in that 20 mM of piperazine is used as the buffer.

4. Process according to claim 1, characterised in that the eluate has a pH in the range from 4.7 to 6.

5. Process according to claims 1-4, characterised in that the resultant immunoglobulin preparation contains 5-100 wt.% IgM, related to the total immunoglobulin content.

6. Process according to claims 1-4, characterised in that the resultant immunoglobulin preparation contains 10 to 35 wt.% IgM and 10 to 35 wt.% IgA, related to the total immunoglobulin content.

7. Process according to one of claims 1 to 6, characterised in that the resultant immunoglobulin preparation is in the form of a solution with a protein concentration of 1 to 20 g/100 ml, preferably 3 to 5 g/100 ml.

8. Process according to claims 1 to 7, characterised in that the resultant immunoglobulin preparation additionally contains proteins, preferably human albumin, sugars, preferably glucose, or amino acids.

9. Process for the production of immunoglobulin preparations according to claims 1 to 4, characterised in that, before or after treatment with the anion exchanger, the solution is optionally heated for 1 minute to 24 hours, preferably for 20 to 40 minutes, to 40 to 60°C, preferably 50 to 54°C.

10. Process for the production of immunoglobulin preparations according to claims 1 to 4, characterised in that, before or after treatment with the anion exchanger, the solution is optionally incubated for 1 minute to 24 hours at pH 3.5 to 5, preferably pH 4 to 4.5.

11. Process according to one of claims 1 to 10, characterised in that polymers bearing TMAE groups are used as the anion exchanger.

12. Process according to one of claims 1 to 10, characterised in that polymers bearing QMA groups are used as the anion exchanger.

13. Process according to one of claims 1 to 10, characterised in that a copolymer prepared from the primary monomer N-acryloyl-2-amino-2-hydroxymethyl-1,3-propanediol and a trimethylaminomethyl derivative of the monomer or neutral polysaccharides with quaternary amino groups is used as the anion exchanger.

14. Process according to one of claims 1 to 10, characterised in that polymers bearing DEAE groups are used as the anion exchanger.

15. Process according to claim 14, characterised in that a copolymer prepared from the primary monomer N-acryloyl-2-amino-2-hydroxymethyl-1,3-propanediol and a diethylaminomethyl derivative of this monomer is used as the anion exchanger.

16. Process for the production of immunoglobulin preparations according to one of claims 1 to 15, characterised in that, before or after treatment with the anion exchanger, viruses present in the solution are inactivated with 0.05% β-propiolactone and/or UV irradiation, by treatment with solvents and/or detergents or by pasteurisation in the solution.

## Revendications

1. Procédé de production d'une préparation d'immunoglobulines poly-clonales administrable par voie intraveineuse, non modifiée chimiquement, contenant plus de 5 % en masse d'IgM et/ou plus de 10 % d'IgA, par rapport à la teneur totale en immunoglobulines, et présentant une faible activité anti-complémentaire, caractérisé en ce que l'on traite une fraction de Cohn II/III ou III ou des fractions plasmatiques contenant des IgA et/ou IgM équivalentes avec un échangeur d'anions, on élue celui-ci avec un gradient de chlorure de sodium avec un tampon dans le domaine acide de telle manière que les protéines ayant une activité anti-complémentaire supérieure à 50 CH50/ml pour 50 g d'immunoglobulines par litre de solution restent liées et que les immunoglobulines de l'éluat présentent une activité anti-complémentaire inférieure à 50 CH50/ml pour 50 g d'immunoglobulines par litre de solution.

2. Procédé selon la revendication 1, caractérisé en ce que le gradient de chlorure de sodium présente une concentration de 20 à 175 mM.

3. Procédé selon la revendication 1, caractérisé en ce que l'on utilise comme tampon de la pipérazine 20 mM.

4. Procédé selon la revendication 1, caractérisé en ce que l'éluat présente un pH dans le domaine de 4,7 à 6.

5. Procédé selon les revendications 1 à 4, caractérisé en ce que la préparation d'immunoglobulines produite comporte 5 à 100 % en masse d'IgM par rapport à la teneur totale en immunoglobulines.

6. Procédé selon les revendications 1 à 4, caractérisé en ce que la préparation d'immunoglobulines produite contient 10 à 35 % en masse d'IgM et 10 à 35 % en masse d'IgA par rapport à la teneur totale en immunoglobulines.

7. Procédé selon l'une des revendications 1 à 6, caractérisé en ce que la préparation d'immunoglobulines produite est sous forme d'une solution ayant une concentration en protéines de 1 à 20 g/100 ml, de préférence de 3 à 5 g/100 ml.

8. Procédé selon les revendications 1 à 7, caractérisé en ce que la préparation d'immunoglobulines produite contient en outre des protéines, de préférence de l'albumine humaine, des sucres, de préférence du glucose, ou des acides aminés.

9. Procédé de production de préparations d'immunoglobulines selon les revendications 1 à 4, caractérisé en ce que l'on chauffe la solution pendant 1 min à 24 h, de préférence pendant 20 à 40 min, entre 40 et 60°C, de préférence entre 50 et 54°C, éventuellement avant ou après le traitement avec l'échangeur d'anions.

10. Procédé de production de préparations d'immunoglobulines selon les revendications 1 à 4, caractérisé en ce que l'on incube la solution pendant 1 min à 24 h à un pH de 3,5 à 5, de préférence à un pH de 4 à 4,5, éventuellement avant ou après le traitement avec l'échangeur d'anions.

11. Procédé selon l'une des revendications 1 à 10, caractérisé en ce que l'on utilise comme échangeur d'anions des polymères portant des groupes TMAE.

12. Procédé selon l'une des revendications 1 à 10, caractérisé en ce que l'on utilise comme échangeur d'anions des polymères portant des groupes QMA.

13. Procédé selon l'une des revendications 1 à 10, caractérisé en ce que l'on utilise comme échangeur d'anions un copolymère constitué par le monomère primaire N-acryloyl-2-amino-2-hydroxyméthyl-1,3-propanediol et par un dérivé triméthylaminométhylé du monomère ou des polysaccharides neutres à groupes aminés quaternaires.

14. Procédé selon l'une des revendications 1 à 10, caractérisé en ce que l'on utilise comme échangeur d'anions des polymères portant des groupes DEAE.

15. Procédé selon la revendication 14, caractérisé en ce que l'on utilise comme échangeur d'anions un copolymère constitué par le monomère primaire N-acryloyl-2-amino-2-hydroxyméthyl-1,3-propanediol et par un dérivé diéthylaminométhylé de ce monomère.

16. Procédé de production de préparations d'immunoglobulines selon l'une des revendications 1 à 15, caractérisé en ce que, avant ou après le traitement avec l'échangeur d'anions, les virus présents dans la solution sont inactivés avec 0,05 % de β-propiolactone et/ou irradiation UV, par traitement avec des solvants et/ou des détergents ou par pasteurisation en solution.
